# EUROPEAN PATENT APPLICATION

(11) **EP 0 976 403 A1**
(43) Date of publication of application: **02.02.2000**
(21) Application number: 98202568.6
(22) Date of filing: 30.07.1998
(51) Int. Cl.: A61K 39/39

(54) **Adjuvant comprising pulmonary surfactant**

(71) Applicant: Stichting Instituut voor Dierhouderij en Diergezondheid (ID-DLO), 8219 PH Lelystad (NL); STICHTING VOOR DE TECHNISCHE WETENSCHAPPEN, 3527 JP Utrecht (NL); NEDERLANDSE ORGANISATIE VOOR TOEGEPAST-NATUURWETENSCHAPPELIJK ONDERZOEK TNO, 2628 VK Delft (NL); Vrije Universiteit Amsterdam, 1081 BT Amsterdam (NL)
(72) Inventor: van Iwaarden, Johan Frederik, 1191 HJ Ouderkerk aan de Amstel (NL); Kraal, Georg, 2012 BM Haarlem (NL)
(74) Representative: Smulders, Theodorus A.H.J., Ir.

(57) **Abstract**

The invention relates to the field of immunology, more specifically to adjuvants used to increase the immunogenicity of antigens, e.g. for incorporation in vaccines. The invention provides use of a pulmonary surfactant or a component thereof as adjuvant substances that are able to stimulate an immune response but that in general are homologous to substances native to the body.

## Description

The invention relates the field of immunology, more specifically to adjuvants used to increase the immunogenicity of antigens, e.g. for incorporation in vaccines. Current adjuvants used in immunology comprise a variety of substances, such as bacterial cell wall components or even whole bacteria (e.g BCG, in complete Freund's adjuvant), mineral oils (e.g in (in)complete Freund's adjuvant), metal salts (e.g. aluminium hydroxide), blockpolymers and others that are in general mixed with an antigen against which an immune response is wanted. Most adjuvants are in general foreign (non-self) to the body, and in general function by causing a local inflammatory response, drawing cells of the immune system to the location of the antigen-adjuvant complex. By virtue of the (enduring) attraction of such cells (such as B- and T-lymfocytes or antigen presenting cells) to the site where the relevant antigen is also present, adjuvants, besides causing an aspecific response, also enhance the induction of a specific immune response directed against said antigen. An intrinsic disadvantage of current adjuvants lies in the local inflammation caused by the immune response to the non-self adjuvant. Such an inflammation can vary from on the one hand very small and harmless, to on the other hand, painfull and detrimental to the vaccinated person or animal. As a consequence, vaccines comprising adjuvants can only be applied to tissue, such as connective tissue or fat or muscle tissue (intra-muscularly), where an inflammation can stay contained and can cause relatively little harm. Other areas where one preferably might want to vaccinate, such as mucosal surfaces or the peritoneal cavity, are not suitable for vaccines comprising above adjuvants, despite the need for for example mucosal vaccination. In addition, most adjuvants, such as those containing mineral oils or metal salts, remain in place at the injection site even long after the antigen has disappeared. The remains and a surrounding tissue reaction can be cumbersome or painful. In addition, such remains are more and more unwanted in animal husbandry. Remaining adjuvant, often encapsulated in an abcess, is often found in the meat of farm animals at slaughter, and invariably needs to be removed before the meat can be sold to customers. For example, in the pig industry, where vaccination is widely and repeatedly practiced, Freund adjuvans is generally not applied, and mineral oil adjuvants are more and more disliked for the above described effects, whereas other types of adjuvants are not as effective. Therefore, new adjuvants are wanted.

The invention provides an adjuvant derivable from a substance capable of inducing self-tolerance in an immune competent animal. Such a substance is in general native to the body of said animal and does not elicit detrimental or unwanted inflammatory immune responses. Since 1900, the central dogma of immunology has been that the immune system does not normally react to self. However, it as recently become apparent that auto-immune responses are not as rare as once thought and that not all auto-immune responses are harmful; some responses play a distinct role in mediating the immune response in general. For example, certain forms of auto-immune response such as recognition of cell surface antigens encoded by the major histocompatibility complex (MHC) and of anti-idiotypic responses against self idiotypes are important, indeed essential, for the diversification and normal functioning of the intact immune system.

The mechanisms of (self)-tolerance induction and the means by which immunological reactivity develops against antigens in immune competent animals (including humans) can be seen as an intricate system of checks and balances which is maintained between various subsets of cells (i.e. T-cells) of the immune system, thereby providing the individual with an immune system capable of developing and maintaining self-tolerance irrespective from its capacity to cope with antigens present on foreign invaders.

In a preferred embodiment the invention provides an adjuvant derivable from a substance capable of inducing self-tolerance in an immune competent animal, wherein said substance comprises pulmonary surfactant or an analogue thereto or a component derived thereof. A pulmonary surfactant or analogue thereof or a component thereof as described herein relates to surfactant proteins and to surfactant lipids and carbohydrates as can be found in the lungs and intestines of animals, including humans. Pulmonary surfactant, which lines the alveoli of the lung of animals, is a complex mixture of lipids, proteins and carbohydrates. A analogue of pulmonary surfactant can be found in the intestines where it forms an essential part of the mucus layer and where it is processed along the lines described below for pulmonary surfactants.

Approximately 90% of surfactant are lipids, 10% are specific proteins and very small amounts are carbohydrates. The lipids are largely phospholipids (70%) with some triacylglycerols and cholesterol. Although surfactant contains no unique lipids, the composition of the phospholipids in surfactant is characteristic for surfactant and well conserved in different species. The major phospholipids in surfactant are phosphatidylcholine (70%) and the negatively charged phosphatidylglycerol (12%).

The invention provides use of a pulmonary surfactant or a component thereof as adjuvant substances that are able to stimulate an immune response but that in general are homologous to substances native to the body and thus lack above described side effects. The invention provides the use of substances that are related to or associated with pulmonary surfactant proteins as adjuvant in mixtures with antigens, such as vaccines, thereby providing immunogenic substances and vaccines for use with animal and man.

The invention also provides the use of an adjuvant derivable from a substance capable of inducing self-tolerance in an immune competent animal, such as surfactant protein or an analogue thereof or component thereof, and lipid as adjuvant and provides such an adjuvant. Lipids can comprises lipid-like substances related to those found in pulmonary surfactant but can also comprises fatty acid or derivatives thereof having preferably a hydrofobic nature and low or none antigenicity. A preferred embodiment of the invention is provided by use in said adjuvant as provided by the invention of surfactant protein A, B, C and or D, or an analogue such as a hydrophobic peptide derived thereof as adjuvant. Until thus far four surfactant specific proteins have been described. Two hydrophilic proteins, surfactant protein A (SP-A) and surfactant protein D (SP-D), and two hydrophobic proteins, surfactant protein B (SP-B) and surfactant protein C (SP-C). Pulmonary surfactant, i.e. proteins and lipids, are synthesised by alveolar type II cells. SP-A, SP-B and SP-D are also produced by the broncheolar CLARA cells as well as cells in the intestines (SP-A and SP-B) or stomach (SP-D). After synthesis in the alveolar type II cells pulmonary surfactant is packed into a secretory granule, the lamellar body. Following the secretion of surfactant by the type II cells several different lipid conformations are formed which seem to have their own particular functions. These forms are: a) Lattice-like structures (tubular myelin), b) densely packed multilamellar structures, c) multilamellar and unilamellar structures and d) a thin surface film.

It is hypothesised that tubular myelin which can be formed from phosphatidyl choline, phosphatidyl glycerol, SP-A, SP-B and calcium, is the precursor of the surface film which covers the alveoli of the lung. The surface film is for example responsible for the lowering of the surface tension of the alveoli thereby preventing alveolar collapse at end-expiration. Whether tubular myelin is the only precursor of the surface film is not clear, since small liposomes consisting of phosphatidyl choline, phosphatidyl glycerol and SP-B can readily insert into the surface film *in vitro*.

After functioning as a surface reducing agent, surfactant must be removed from the alveoli as there is active secretion and a constant pool size of surfactant. The structural form of surfactant leaving the alveolus is largely unknown, it possibly is a fraction rich in small vesicles. Although the alveolar macrophages degrade some surfactant and small amounts of surfactant may exit the alveolar spaces via the airways and the circulation, the major cells which internalise surfactant are the alveolar type II cells. It is postulated that SP-A as well as SP-B may be involved in the recycling of the surfactant phospholipids, since both proteins were demonstrated to enhance the uptake of lipids by alveolar type II cells *in vitro*.

The invention also provides an immunogenic substance or vaccine comprising antigen and an adjuvant according to the invention. For example, the invention provides an immunogenic substance or vaccine comprising antigen and surfactant protein or an analogue thereof and optionally further comprising (surfactant) lipids. Without doubt the major function of pulmonary surfactant is the reduction of the surface tension in order to maintain the integrity of the alveoli. However, there is accruing evidence that the pulmonary surfactant system also plays an important role in the defence against airborne pathogens in the lung, and similarly intestinal surfactant is involved in maintaining the mucosal immune defence in the intestines. For instance, SP-A was demonstrated to enhance the phagocytosis of bacteria and viruses by alveolar macrophages and to inhibit the infection of cells by influenza viruses. In addition, the alveolar type II cells were shown to produce a wide variety of proinflammatory cytokines and chemokines including IL-3, IL-6, GM-CSF, MCP-1 and RANTES. Besides non-specific immune defences, as described above, the lung and intestines also have a specific immune system comprising Bronchus Associated Lymphoid Tissue (BALT) or Gut Associated Lymphoid Tissue (GALT) and draining lymph nodes. In these lymphoid organs specific T cells are formed and the production of antibodies can take place. However, under normal conditions the immune system in the lung and gut is suppressed. Only replicating micro-organisms evoke an immune response which consists primarily of specific antibodies of the IgA class. However, as shown in the experimental part, substances analogous to surfactant proteins or lipids, when mixed with antigen, can target this antigen to antigen presenting cells or can cause uptake or presentation of antigen by antigen presenting cells, resulting in an immune response against the antigen.

As a preferred embodiment of the invention is provided an immunogenic substance or vaccine comprising antigen and surfactant protein or an analogue thereof and further comprising a liposome. An advantage for the use of surfactant and liposome is that these are naturally occurring in the form of surfactant and that surfactant (porcine, bovine) comprising liposomes, in the form of surfactant, have already been used to treat premature children to achieve better lung function, showing no side effects related to the liposomes.

Surfactant-antigen-immune cell interaction, which is possibly akin to the processes that find place during reuptake of surfactant molecules under physiological conditions by alveolar type II cells and macrophages, allows the specific and selective generation of an immune response against the antigen mixed with substance analogous to surfactant. However, since (modified) surfactant proteins of various species show considerable homology, these substances are not or only barely recognised by the body as non-self (they actually are capable of inducing self-tolerance in the immune competent animal, as most if not all substances native to the animal's body) and will not cause the aspecific inflammmatory responses so characteristic for the above indicated adjuvants. Thus, although these substances are actively involved in generating a specific immune response against the antigen of choice, no detrimental side effects such as aspecific inflammatory responses as found with the above described classical adjuvants will be generated. In addition, these substances will finally be cleared from the injection site, by mechanisms routinely applied for example during processing of surfactant in the lung, and will not leave remains at the injection site.

A vaccine or immunogenic substance provided by the invention can be applied via various vaccination routes known in the art. A preferred application is intramuscular vaccination, but also other application routes, such as intratracheal, ocular, oral, nasal or aerosol application are feasible.

The invention is further explained in the experimental part of this description which is not limiting the invention.

### Experimental part

### Surfactant proteins.

Until thus far four surfactant specific proteins have been described. Two hydrophilic proteins, surfactant protein A (SP-A) and surfactant protein D (SP-D), and two hydrophobic proteins, surfactant protein B (SP-B) and surfactant protein C (SP-C). The most abundant protein in surfactant is SP-A (more than 50%). SP-A consists of 28 to 35 kDa peptides assembled into a large multimeric protein composed of 18 similar subunits. Also SP-D is a large multivalent molecule comprising 12 monomers of 39 to 43 kDa peptides. Both proteins have a collagen-like sequence and a COOH-terminal Ca²⁺-dependent carbohydrate binding domain. They belong to the C-type mammalian lectins. The surfactant proteins B and C are extremely hydrophobic proteins which in general are dissolved in organic solvents. SP-B is a dimer which consists of two 8 kDa peptides whereas SP-C is a monomer with a molecular weight of 8 kDa, containing one (canine) or two (human and pig) palmitoyl groups.

The suppression of the immune-system in the lungs and gut is mediated by (alveolar) macrophages. In mice after depletion of their alveolar macrophages by intratracheal instillation of clodronate containing multilamelar liposomes, followed by intratracheal instillation of antigen, a local immune response against the antigen can be detected. Hence it seems feasible to develop vaccination protocols for vaccination via for example the airways comprising depletion of alveolar macrophages by clodronate containing liposomes, and intratracheal insertion of antigen.

Antigen, TNP-KLH, was entrapped in liposomes consisting of lipids and various concentrations of surfactant proteins and intratracheally instilled in mice which were either depleted of their alveolar macrophages or in control mice. Subsequently, the primary immune response against the antigen in these mice was determined after isolation of their spleen cells using an ELI-spot assay. In addition, the secondary immune response was measured in mice which were i.p. injected with antigen after the instillation of antigen containing liposomes, in their sera using ELISA's specific for IgG, IgM and IgA.

### MATERIAL & METHODS

### Animals

Young adult (8-12 weeks, female) BALB/c mice were obtained from Harlan CPB (The Netherlands).

### Surfactant proteins and lipids

SP-A was isolated from bronchoalveolar lavage fluid of patients with alveolar proteinosis as described (1). SP-A was dissolved in 5 mM HEPES pH 7.4 (2.2 mg/ml) and stored in small aliquots at -70°C. SP-B and SP-C were isolated from porcine lung lavage. Porcine lungs were obtained from the slaughterhouse and lavaged 3-5 times with a solution of 154 mM NaCl.

Pulmonary surfactant was prepared from the bronchoalveolar lavage by the method of Hawgood et a1 (2). Lung surfactant was extracted with 1-butanol (1). Butanol was dried by rotary evaporation, and the residue was dissolved in chloroform/methanol/0.1 M HC1 (1:1:0.05, v/v/v). Insoluble material was removed by centrifugation. SP-B and SP-C were separated from lipids and purified to homogeneity by Sephadex LH-60 (Pharmacia, Uppsala, Sweden) chromatography as described (3). The proteins were stored in a mixture of chloroform/methanol (1:1; v/v) at-20°C. The concentration of the proteins was determined by quantitative amino acid analysis. The LH-60 column fractions which contained the surfactant lipids were dried by rotary evaporation. The lipids were dissolved in chloroform/methanol (1:1; v/v). The phospholipid concentration was estimated by determining the phosphorus concentration as described by Bartlett (4).

### Lipids

Dipalmitoylphophatidylcholine (DPPC), phosphatidylcholine (eggPC), phosphatidylglycerol (eggPG) and cholesterol were purchased from Avanti polar lipids (Alabaster, AL, USA).

### Alveolar macrophage-depleting liposomes

86 mg eggPC and 8 mg cholesterol were dissolved in 10 ml chloroform. The chloroform was dried by rotary evaporation. The lipid film was suspended in either 4 ml PBS (control liposomes) or 10 ml dichloromethylene-diphosphonate (DMDP) in PBS (0.189 g/ml). The suspension was kept for 2 hours at room temperature. Subsequently, it was sonicated for 3 min in a waterbath sonicator and kept at room temperature for 2 hours. The DMDP-containing liposomes were suspended in 100 ml PBS and centrifuged at 15,000 g to remove free DMDP. The pellet was suspended in 4ml PBS.

### Antigen-containing liposomes

Lipids were dissolved in the presence or absence of SP-B and/or SP-C in chloroform. The chloroform was dried by rotary evaporation. The lipid film was suspended in 0.8 mg Trinitrophenyl-Keyhole Limpet Hemocyanin (TNP-KLH)/ml PBS by vortexing. Subsequently, the liposomes were sonicated for 1 min on ice using an MSE ultrasonic disintegrator. For the experiments with SP-A, SP-A was either added to the lipid suspension together with the antigen or after the preparation of the antigen-containing liposomes.

### Administration of liposomes

The mice were fixed in an upright position under anaesthetisation with 20 µl of a 4:3 (v/v) mixture of Aescoket (Aesculaap, Gent, Belgium) and Rompun (Bayer, Leverkussen, Germany), intramusculary injected. Using a nylon tube, connected to an 1 ml syringe, 100 µl liposomes were injected through the glottis into the trachea.

### Immunisation procedures

At day 0, mice were intratracheally instilled with 100 µl of either PBS or DMDP-liposomes or PBS liposomes. At day 2, mice were intratracheally injected with 100 µl of antigen-containing liposomes. To study the primary immune response, animals were autopsied at day 9 and their spleen cells were isolated to determine the number of TNP-antibody secreting cells using a SPOT-ELISA. Animals used to study the secondary immune response were injected intratracheally with the same suspensions at the same times as the mice used for the primary response, but were injected with 100 µl TNP-KLH (0.8 mg/ml PBS) intraperitonealy at day 23. The animals were sacrificed at day 30, and their sera were collected in order to determine the TNP-antibody titers using an ELISA.

### SPOT-ELISA

This assay was based on the method described by Sedgwick and Holt (5). Briefly, microtiter plates were coated overnight at 4°C with 5 µg per well trinitrophenylated ovalbumin (TNP-OVA). The plates were emptied and incubated with 1% (w/v) bovine serum albumin (BSA)/ml PBS for 1 h at room temperature. The plates were rinsed twice with PBS. A single cell suspension of spleen cells (10⁷/m1 RPMI 1640 supplemented with 1% HEPES (w/v), 1% BSA (w/v) and 10% fetal calf serum (v/v) was added to the first well of each row (150 µl/well) and serial diluted 1:2 in the same medium. After incubation at 37°C, the wells were rinsed with 0.1% tween 20 (v/v) in PBS (PBT) till the cells were lysed. Subsequently, goat anti mouse IgM (1:500; in 1% BSA-PBT; Sanbio) conjugated with alkaline phosphatase was added to each well (100 µl/well) and the plates were incubated for 2 h at 37°C. After rinsing the plates four times, the alkaline phosphatase substrate, 5-Bromo-4-chloro-3-indolyl-phosphate (1 mg/ml AMP buffer (Sigma) supplemented with 1% low melting agarose (w/v) was added to the wells (100 µl/well) at 37°C. The plates were incubated overnight at 4°C and the cells secreting TNP antibodies were counted.

### ELISA

In order to detect specific antibodies against TNP in the sera of mice, an ELISA was developed based on the method described by Delemarre et al (6). In short, microtiter plates were coated with TNP-OVA and incubated with 1% BSA as described above. The plates were rinsed four times with PBT. For the determination of the IgM and IgG in the sera, the sera were diluted with PBT supplemented with 1% BSA (1:7). In order to measure IgA, the sera were diluted four times with same buffer. The diluted sera were added to the first well of each row (200 µl/well) and serially diluted 1:1 in the PBT-BSA buffer. After incubation of the plates for 2h at room temperature, the plates were rinsed four times with PBT. To each well 100 µl of either biotinylated anti IgM, or anti IgG or anti IgA was added. The plates were incubated for 1 h at room temperature and rinsed four times with PBT. The plates were incubated for 1 h at room temperature avidin conjugated HRP (1:5000 in PBT-BSA), washed and the bound antibodies were visualised by incubation for 1 h at room temperature with the substrate o-phenylenediaminedihydrochloride (2mg/ m1 0.1 M phosphate-citrate buffer pH 5.5 supplemented with 0.015% H₂O₂; 100 µl/well). The reaction was terminated by adding 50 µl of 2.5 M H₂SO₄. The absorbance at 492 nm was measured and the values were expressed as compared to positive control sera for either IgM, or IgG or IgA. The control sera were included on each plate, and were raised in mice by immunisation with TNP-OVA.

### RESULTS

### Entrapment of antigen in surfactant liposomes

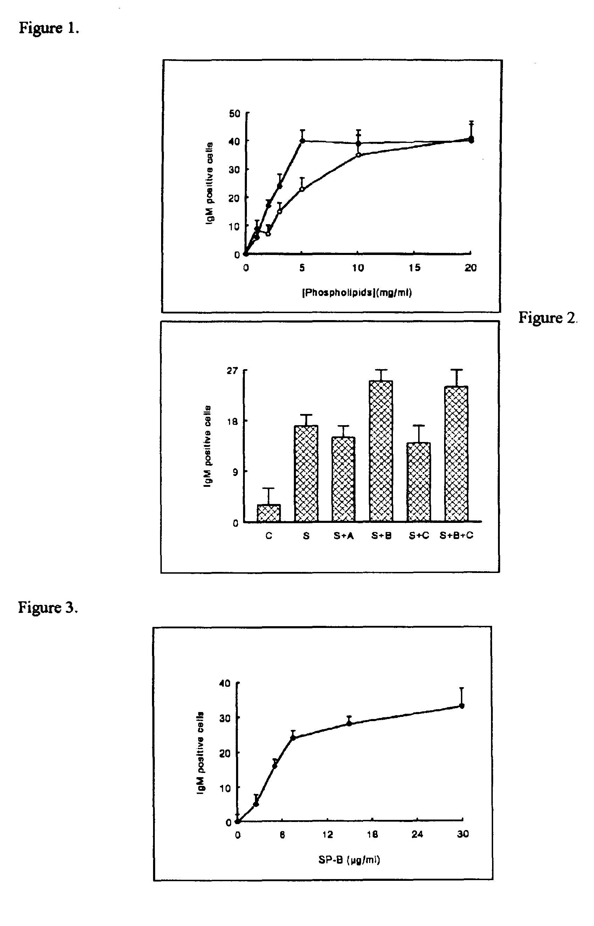

Depletion of AM by DMDP containing liposomes followed by intratracheal instillation of antigen incorporated in small unilamellar vesicles (SUV) leads to the production and secretion of specific IgM antibodies in the spleen cells of mice (Fig 1). The mice remained healthy throughout the experiment and no inflammatory responses were observed in the lungs of the treated mice. A maximal immune response is obtained at lower phospholipid concentrations, 5 mg/ml versus 20 mg/ml, when TNP-KLH is incorporated in SUV's which contain the surfactant lipids and also the surfactant proteins SP-B and SP-C compared to SUV's which consists of only the surfactant lipids. However, at high lipid concentrations, 20 mg/ml, no difference is observed between SUV's containing the surfactant proteins and SUV's devoid of the hydrophobic proteins. The half maximal immune response for the surfactant SUV's which contained SP-B and SP-C was observed at a phospholipid concentration of 3 mg/ml. This concentration was used in the following experiments.

### The effect of the different surfactant proteins on the immune response

In order to determine which surfactant protein(s) entrapped in antigen surfactant liposomes can enhance the immune response, the following experiment was performed. Antigen liposomes were prepared which consisted of the surfactant lipids, and the various surfactant proteins. The lipid to protein ratio was approximately the same as the physiological ratio, i.e. for SP-A: phospholipid/SP-A (15:1(w/w), for SP-B: phospholipid/SP-B (100:0.5-1(w/w) and for SP-C: phospholipid/SP-B (100:0.5-1) (3,7). The antigen liposomes which contained SP-B yielded a higher immune response than the other liposomes (Fig 2). The liposomes which contained only SP-C of SP-A gave a similar immune response as the liposomes which consisted of the surfactant lipids. In addition, incorporation of SP-C in SP-B containing antigen liposomes did not influence the SP-B mediated enhancement of the immune response. In this experiment SP-A was encapsulated in the antigen liposomes together with the antigen, when SP-A was added to the antigen-liposomes, i.e. was not present in the liposomes, similar results were obtained (results not shown).

### SP-B-mediated enhancement of the immune response

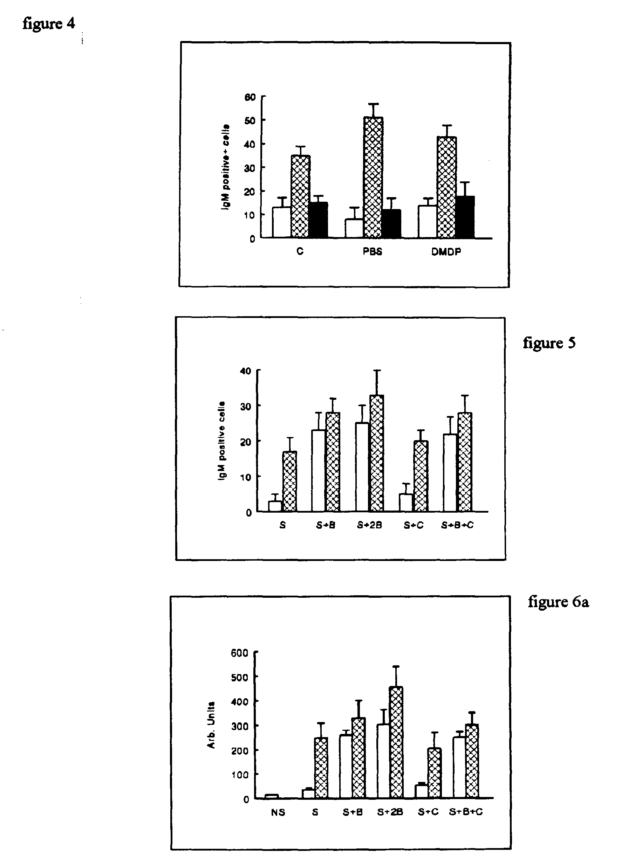

To determine the concentration dependency of the SP-B induced enhancement of the immune response, various concentrations of SP-B were incorporated in antigen containing liposomes. An optimal immune response was observed as a phospholipid to protein ratio of 200:1 (3 mg phospholipid: 15 µg SP-B) (Fig 3). For stimulation of the immune response by antigen SP-B liposomes, the presence of the negatively charged phospholipid, phosphatidylglycerol (PG), in the liposomes is required (Fig 4). Since no effect of SP-B inclusion in the antigen-liposomes on the immune response was noted when the antigen-liposomes consisted of a lipid mixture of DPPC, eggPC, cholesterol (61:30:9; w/w/w) whereas the SP-B activity was restored when a mixture of the lipids DPPC, eggPC, cholesterol and PG (55:27:8:10; w/w/w) was used.

### Influence of AM depletion on the SP-B mediated stimulation of the immune response

In order to determine if depletion of AM by intratracheal instillation of DMDP-containing liposomes is needed to elicit an immune response via the airways when antigen SP-B liposomes are used, the following experiment was performed. Mice were divided in three groups, group 1 was intratracheally instilled with only the antigen containing liposomes, group 2 was intratracheally injected with PBS liposomes followed two days later by the antigen liposomes, and group 3 received the DMDP liposomes and the antigen liposomes (Fig 4). The use of PBS-containing liposomes instead of DMDP-containing liposomes or no liposome treatment at all prior to the intratracheal instillation TNP-KLH encapsulated in the SP-B-DPPC/PC/cholesterol/PG liposomes, yields an immune response in the spleen cells which is approximately the same as when the AM were depleted, Also when surfactant lipids were used instead of the DPPC/PC/cholesterol/PL lipid mixture in combination with the hydrophobic surfactant proteins for antigen liposomes, similar results were obtained (Fig 5).

### Influence of SP-B containing antigen liposomes on the secondary immune response

In these experiments, mice were either intracheally injected with DMDP-liposomes or not on day 0. At day 2, mice received intratracheal antigen-liposomes of different composition. At day 23, mice were boosted with an ip injection of antigen. At day 30, mice were sacrificed and the sera were collected to determine the titers of specific antibodies against the antigen as well as their classes.

A high IgM titer was detected in the sera of mice which were not depleted of their AM, intratracheally primed with SP-B containing antigen liposomes, and boosted with antigen (Fig 6a).

Additional depletion of AM caused only a slight enhancement of the IgM titers. In contrast, only a poor IgG titer is observed when the animals were not depleted of their AM with marginal effects of incorporation of SP-B in the antigen liposomes (Fig 6b). Higher IgG titers are observed when the mice were depleted of their AM prior to the intratracheal instillation of antigen liposomes, which can be further increased by the use of SP-B containing antigen liposomes.

### DISCUSSION

Antigen entrapped in surfactant liposomes can induce a systemic immune response in mice, as shown after intratracheal instillation. No depletion of the alveolar macrophages by an intratracheal injection of DMDP-containing liposomes is needed to observe this immune response. A preferred method for the induction of the immune response via the airways is a method whereby the antigen is entrapped in liposomes which contain at least SP-B and phosphatidylglycerol. The SP-B effect is concentration dependent reaching a maximum at the physiological phospholipid: SP-B ratio of 100:0,5-1. The observed PG dependency of SP-B is not surprising, since the positive charges of SP-B were reported to be essential for its activity (8) such as the phospholipid insertion in the surface film, which covers the alveoli, via the negatively charged PG (9,10).

In a previous study from our laboratory, Thepen et al (11) demonstrated that only a local, IgA, immune response can be elicited in the lung after prior depletion of the alveolar macrophages by DMDP-containing liposomes. Depletion of alveolar macrophages was necessary to induce an immune response via the airways, because alveolar macrophages were shown to inhibit immune responses probably via the suppression of T-cells and dendritic cells.

Depletion of alveolar macrophages which leads to the destruction of cells which may compete with the type II cells for the uptake of antigen containing liposomes as well as a reduction of the suppression of the immune response, is no longer needed to induce a primary immune response when animals are intratracheally injected with SP-B-containing antigen liposomes.

### Applications of SP-B containing liposomes

The advantages for the use of SP-B containing liposomes are: a. naturally occuring in the form of surfactant, b. the homology of SP-B derived from different species (canine, human, porcine rabbit) is high (more than 80%), c. SP-B (porcine, bovine) containing liposomes, in the form of surfactant, have already been used to treat premature children.

### Applications

1) SP-B containing liposomes can be used, as in this study, for immunisation via the airways. A systemic immune response, IgM, can be obtained. No depletion necessary.
2) In combination with AM depleting liposomes, SP-B-antigen liposomes can be used for immunisation.
3) Since SP-B and surfactant analogue material is present in the intestines, immunisation via the intestines using SP-B containing antigen liposomes is effective.
4) In the lungs or in the intestines SP-B containing liposomes can be used to transport the following substances to the alveolar type II cells (or similar cells in the intestines):
   a) DNA, SP-B coupled to modified SP-B has been shown to enhance transfection. Probably SP-B, DNA containing liposomes may work to get DNA in vivo into the type II cells.
   b) transport of corticosteroids to alveolar type II cells. These substances are used for immature children in order to induce the surfactant production by type II cells.
   c) transport of antioxidants to type II cells. Has been shown for SP-A containing liposomes.

### REFERENCES

1. Haagsman HP, Hawgood S, Sargeant T, Buckley D, White RT, Drickamer K, Benson BJ. 1987. The major lung surfactant protein, SP28-36, is a calcium-dependent, carbohydrate binding protein. *J. Biol. Chem*. 262:13877-13880.
2. Hawgood S, Benson BJ, Hamilton, RL. 1985. Effects of a surfactant-associated protein and calcium ions on the structure and surface activity of lung surfactant lipids. *Biochemistry* 24: 184-190.
3. Oosterlaken-Dijksterhuis MA, van Eijk M, van Buel BLM van Golde LMG, Haagsman HP. 1991. Surfactant protein composition of lamellar bodies isolated from rat lung. *Biochem.J*. 274: 115-119.
4. Bartlett, GR. 1959. Phosphorous assay in column chromatography. *J. Biol*. *Chem.* 234:466-468.
5. Sedgwick JD, Holt PGA. 1983. Solid-phase immunoenzymatic technique for the enumberation of specific antibody-secreting cells. *J. Immunol.Meth.* 57:301-309.
6. Delemarre FGA, Claassen E, van Rooijen N. 1989. The primary *in situ* immune response in popliteal lymph nodes and spleen of mice after subcutaneous immunisation with thymus-dependent or thymus-independent (type 1 and 2) antigens. *Anat.Rec*. 223:152-157.
7. Phizackerley PJR, Town MH, Newman GE. 1979. Hydrophobic proteins of lamellated osmiophilic bodies isolated from pig lung. *Biochem J*. 183:731-736.
8. Cochrane CG, Revak SD. 1991. Pulmonary surfactant protein (SP-B): structure-function relationships. *Science*. 253:566-568.
9. Yu SH, Possmayer F. 1992. Effect of pulmonary surfactant protein B (SP-B) and calcium on phospholipid adsorption and squeeze-out of phosphatidylglycerol from binary phospholipid monolayers containing dipalmitoylphosphatidylcholine. *Biochim. Biophys. Acta*. 1126:26-34.
10. Yu SH, Possmayer F. 1992. Studies on surfactant-associated protein B mediated adsorption of surfactant phospholipids. *Am.Rev.Respir.Dis*. 145:A874.
11. Thepen T, van Rooijen N. Kraal G. 1989. Alveolar macrophage elimination in vivo is associated with an increase in pulmonary immune response in mice. *J.Exp.Med.* 170:499-509

### LEGENDS

*Figure 1. Surfactant liposomes*.
   BALB/c mice were intratracheally injected on day 0 with DMDP-containing liposomes (100µl/n\mouse). On day 2, mice were either intratracheally injected with surfactant liposomes which contained no surfactant proteins (open circles; 100 µl liposomes suspension/mouse) or with surfactant liposomes which contained the surfactant proteins SP-B and SP-C (closed circles; 100 µl liposomes suspension/mouse). The mice were sacrificed on the day 9, and the number of anti (IgM)-TNP positive cells/10⁶ spleen was determined (n=5, mean +/-SD).
*Figure 2. Influence of the surfactant proteins on the primary immune response*.
   BALB/c mice were intratracheally injected on day 0 with DMDP-containing liposomes (100 µl/mouse). On day 2, mice were intratracheally injected with 100 µl TNP-KLH (=C), or 100 µl TNP-KLH-surfactant lipids containing liposomes (3 mg phospholipids/ml;=S), or 100 µl TNP-KLH surfactant lipids + SP-A containing liposomes (3mg phospholipids/ml; 200 µg SP-A/ml; =S+A), or 100 µl TNP-KLH-surfactant lipids + SP-B containing liposomes (3 mg phospholipids/ml;15 µg SP-B/ml; = S+B), or 100 µl TNP-KLH-surfactant lipids + SP-C containing liposomes (3 mg phospholipids/ml; 15 µg SP-C/ml; =S+C), or 100 µl TNP-KLH-surfactant lipids + SP-B + SP-C containing liposomes (3 mg phospholipids/ml; 15 µg SP-B/ml; 15 µg SP-C/ml; =S+B+C). The mice were sacrificed on the day 9, and the number of anti (IgM)-TNP positive cells/10⁶ spleen cells was determined (n=5,mean+/-SD).
*Figure 3. SP-B concentration dependency*.
   BALB/c mice were intratracheally injected on day 0 with DMDP-containing liposomes (100 µl/mouse). On day 2, mice were intratracheally injected with 100 µl TNP-KLH-surfactant lipids (3 mg phospholipids/ml) + the indicated concentrations of SP-B containing liposomes The mice were sacrificed on the day 9, and the number of anti (IgM)-TNP positive cells/10⁶ spleen cells was determined (n=5,mean+/-SD).
*Figure 4. The influences of AM depletion and the lipid composition on the primary immune response*.
   BALB/c mice were either not disturbed (=C), or intratracheally injected with PBS-containing liposomes (100 µl/mouse;=PBS), or DMDP-containing liposomes (100 µl/mouse;=DMDP). On day 2, mice were intratracheally injected with either 100 µl TNP-KLH-containing liposomes which consisted of the following lipids (3 mg phospholipids/ml; DPPC/eggPC/eggPG/cholesterol; 55:27:10:8 (w/w/w/w) (open bar), or 100 µl TNP-KLH-containing liposomes which consisted of the following lipids (3 mg phospholipids/ml; DPPC/eggPC/eggPG/cholesterol:55:27:10:8 (w/w/w/w) supplemented with 15 µg SP-B/ml (cross hatched bar), or 100 µl TNP-KLH-containing liposomes which consisted of the following lipids (3 mg phospholipids/ml; DPPC/eggPC/cholesterol: 61:30:9 (w/w/w) supplemented with 15 µg SP-B/ml (closed bar). The mice were sacrificed on the day 9, and the number of anti (IgM)-TNP positive cells 10⁶ spleen cells was determined (n=5, mean +/-SD).
*Figure 5. The influences of AM depletion and the surfactant proteins on the primary immune response*.
   BALB/c mice were either not disturbed (=open bar), or intratracheally injected with DMDP-containing liposomes (100 µl/mouse;=DMDP) (=cross hatched bar). On day 2, mice were intratracheally injected with 100 µl TNP-KLH-surfactant lipids containing liposomes (3 mg phospholipids/ml; =S), or 100 µl TNP-KLH-surfactant lipid + SP-B containing liposomes (3 mg phospholipids/mi; 15 µg SP-B/ml; =S+B), or 100 µl TNP-KLH-surfactant lipids + SP-B containing liposomes (3 mg phospholipids/ml; 30 µg SP-B/ml; =S+2B), or 100 µl TNP-KLH-surfactant lipids + SP-C containing liposomes (3 mg phospholipids/ml; 15 µg SP-C/ml; = S+C), or 100 µl TNP-KLH-surfactant lipids + SP-B+SP-C containing liposomes (3 mg phospholipids/ml; 15 µg SP-B/ml; 15 µg SP-C/ml; =S+B+C).The mice were sacrificed on the day 9, and the number of anti (IgM)-TNP positive cells/10⁶ spleen cells was determined (n=5, mean +/-SD).
*Figure 6. The influences of AM depletion and the surfactant proteins on the secondary immune response*.
   BALB/c mice were either not disturbed (=open bar), or intratracheally injected with DMDP-containing liposomes (100 µl/mouse;=DMDP) (=cross hatched bar). On day 2, mice were intratracheally injected as desbribed under legend figure 5, except for NS which were control mice, which were not treated at all. On day 23, all mice, except for the control mice (NS), were injected i.p. with 100 µl TNP-KLH (0.8 mg/ml PBS). At day 30, the mice were sacrificed and their sera collected Figure 6, depicts the IgM titers of the sera compared to a positive control serum, which was included in every plate.

## Claims

1. An adjuvant derivable from a substance capable of inducing self-tolerance in an immune competent animal.

2. An adjuvant according to claim 1 wherein said substance comprises pulmonary surfactant or an analogue thereof or a component derived thereof.

3. An adjuvant according to claim 2 wherein said pulmonary surfactant comprises pulmonary surfactant protein A, B, C and/or D.

4. An immunogenic substance or vaccine comprising antigen and an adjuvant according to anyone of claims 1 to 3.

5. An immunogenic substance or vaccine according to claim 4 and further comprising lipid.

6. An immunogenic substance or vaccine according to claim 4 or 5 and further comprising a liposome.

7. Use of an pulmonary surfactant or an analogue thereof or a component thereof as adjuvant.

8. Use according to claim 7, wherein said pulmonary surfactant comprises pulmonary surfactant protein A, B, C and/or D.
